# EUROPEAN PATENT APPLICATION

(11) **EP 4 321 201 A1**
(43) Date of publication of application: **14.02.2024**
(21) Application number: 21936015.3
(22) Date of filing: 07.04.2021
(51) Int. Cl.: A61M 25/09

(54) **MEDICAL WIRE MATERIAL AND GUIDE WIRE**

(71) Applicant: Asahi Intecc Co., Ltd., Seto-shi, Aichi 489-0071 (JP)
(72) Inventor: KAMAKURA, Nagisa, Seto-shi, Aichi 489-0071 (JP); SATO, Kazufumi, Seto-shi, Aichi 489-0071 (JP)
(74) Representative: Winter, Brandl - Partnerschaft mbB
(86) International application number: PCT/JP2021/014820
(87) International publication number: WO 2022/215211

(57) **Abstract**

An object of the present invention is to provide a wire material for medical use and a guide wire with excellent resilience.

After a strain amount of 2% is applied by bending the wire material so that each end approached each other, a resilience rate is 96% or more. Strain amount (%) = (R/(L-R)) × 100 (R is a wire diameter of the wire material, L is a distance between both ends when the wire material is bent so that each ends approaches each other), and the resilience rate (%) = (1-θ/180) × 100 (θ is an angle at which two tangent lines intersect when the tangent lines are drawn from both ends of the wire material after bending).

## Description

### [Technical Field]

This disclosure relates to a wire material for medical use.

### [Background Art]

When treating a stenosis that occurs in blood vessels such as coronary arteries surrounding the heart, or when treating a region where the blood vessel is completely occluded due to the progress of calcification (for example, chronic total occlusion: CTO), a guide wire for guiding a treatment instrument such as a balloon catheter is inserted into the blood vessel, prior to the treatment instrument.

For example, Patent Literature 1 proposes a guide wire made of SUS304.

### [Citation List]

### [Patent Literature]

[Patent Literature 1] JP 2009-172229 A

### [Summary of Invention]

### [Technical Problem]

Now, regarding the above guide wire, it is known that there is a correlation between the operability of the guide wire and the ability to restore its original shape (resilience) after bending etc. However, no guidewire with excellent resilience has been proposed.

An object of the disclosure is to provide a wire material for medical use and a guide wire with excellent resilience.

### [Solution to Problem]

In order to solve the above problems, a wire material for medical use according to an aspect of the disclosure is a wire material for medical use made of stainless steel. The wire material has a resilience rate is 96% or more after a strain amount of 2% is applied by bending the wire material so that each end approaches each other. The strain amount (%) = (R/(L-R)) × 100 (R is a wire diameter of the wire material, L is a distance between both ends when the wire material is bent so that each end approaches each other), and the resilience rate (%) = (1-θ/180) × 100 (θ is an angle at which two tangent lines intersect when the tangent lines are drawn from both ends of the wire material after bending).

The stainless steel may have tensile strength of 2800 N/mm² or more and less than 3400 N/mm².

The stainless steel may be austenitic stainless steel conforming to ASTM F2581.

When the wire material is bent 180°, breakage does not have to occur at the bent portion.

A guide wire according to one aspect of the disclosure includes the above wire material for medical use and a coil body in which it is provided.

### [Advantageous Effects of Invention]

The disclosure can provide a medical wire material and a guide wire with excellent resilience.

### [Brief Description of Drawings]

FIG. 1 illustrates a schematic cross-sectional view of a guide wire according to an embodiment of the disclosure.
FIG. 2 is a schematic diagram of a jig to be used for resilience evaluation.
FIG. 3 is an explanatory diagram of resilience evaluation.
FIG. 4 is an explanatory diagram of a method for measuring a residual angle.
FIG. 5 is a graph that shows changes in a resilience rate with respect to a strain amount of each wire type.
FIG. 6 is an explanatory diagram of a bending test.
FIG. 7 is a schematic diagram of a second jig to be used for evaluating rotation performance.
FIG. 8 is a graph that shows a relationship between a hand angle and a distal end angle in rotation performance evaluation.
FIG. 9 is a graph that shows the relationship between the hand angle and the distal end angle in rotation performance evaluation.

### [Description of Embodiments]

Hereinafter, one embodiment of the disclosure is described with reference to drawings. However, the disclosure is not limited to only the embodiments illustrated in the drawings.

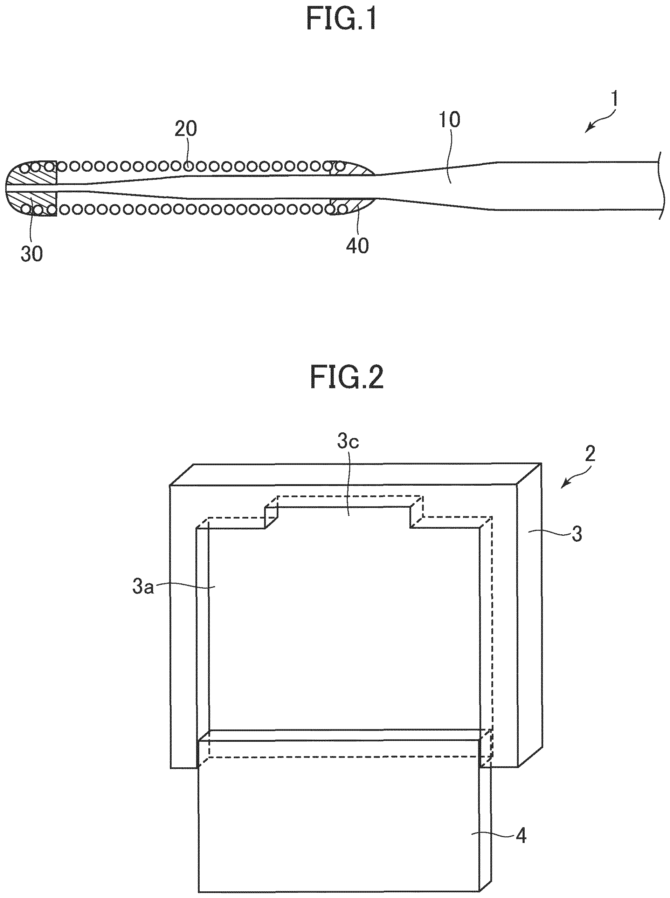

FIG. 1 is a schematic cross-sectional view of a guide wire 1 according to an embodiment of the disclosure.

As illustrated in FIG. 1, the guide wire 1 includes a core shaft 10, a coil body 20, a distal end joint part 30, and a proximal end fixing part 40.

The core shaft 10 has a round bar shape that tapers from a proximal end toward a distal end side. At an end on the proximal end side, a user performs rotational operation etc., of the guide wire 1.

The coil body 20 is formed in a hollow cylindrical shape by spirally winding a single metal wire 21 around the core shaft 10. As materials for the coil body 20, X-ray opaque materials such as gold, platinum, tungsten, or alloys containing these elements, or stainless steel, superelastic alloys, cobalt-based alloys, and the like can also be used.

The distal end joint part 30 constitutes the distal end of the guide wire 1 and has a substantially hemispherical shape. As materials for the distal end joint part 30, metals such as silver, gold, or alloys thereof, lead-free solder, brazing materials, adhesives, or the like can be used.

The proximal end joint part 40 fixes the proximal end of the coil body 20 to the core shaft 10. As materials for the proximal end joint part 40, for example, lead-free solder such as an Sn-Ag alloy, an Sn-Ag-Cu alloy, an Au-Sn alloy, and Au-Ge, or brazing materials are used.

The core shaft 10 is made of stainless steel conforming to ASTM F2581 (C: 0.15 to 0.25% by mass, Mn: 9.50 to 12.50% by mass, P: 0.020% by mass Max, S: 0.010% by mass Max, Si: 0.20 to 0.60% by mass, Cr: 16.5 to 18.0% by mass, Ni: 0.05% by mass Max, Mo: 2.70 to 3.70% by mass, N: 0.45 to 0.55% by mass, Cu: 0.25 mass Max, Fe: Bal.).

In order to produce the core shaft 10, the stainless steel material (base material) is subjected to wire drawing, straightening, and heat treatment.

The wire drawing is not particularly limited as long as it can continuously reduce the wire diameter of a steel material, and may be either a process using dies or a process using rolls. The area reduction rate of a wire material in processing ranges from preferably 80% to 97%, for example. Here, the area reduction rate is defined by (1-d1²/d0²) × 100. Wherein d0 is the wire diameter of a base material (wire material before processing), and d1 is the wire diameter of a drawn wire material (wire material after processing). Straightening is not particularly limited as long as a wire material can be straightened, and may be performed by a combination of processing using a plurality of straightening rollers or stretchers, tension annealing using heat treatment, and the like.

Heat treatment is performed at 300°C to 800°C.

The core shaft 10 is obtained by cutting the wire material after heat treatment and tapering an end portion of the wire material so that the outer diameter gradually decreases toward the distal end. Alternatively, the core shaft 10 may also be produced by wire drawing and heat treatment without straightening.

As described later, the stainless steel wire material that constitutes the core shaft 10 has a resilience rate of 96% or more after a strain amount of 2% is applied by bending the wire material so that the ends approach each other. The stainless steel constituting the core shaft 10 has tensile strength of 2800 N/mm² or more and less than 3400 N/mm².

### [Examples]

Next, various tests conducted to confirm the properties of the stainless steel wire material constituting the core shaft 10 of the disclosure will be described. Table 1 shows the wire types used in the tests.

**[Table 1]**

| | Wire type | Chemical composition (mass%) | | | | | | | | Wire diameter (mm) | Tensile strength (N/mm²) |
|---|---|---|---|---|---|---|---|---|---|---|---|
| | | Cr | Mn | Mo | Si | N | C | Ni | Fe | | |
| Example | A1 | 17.35 | 11.98 | 3.12 | 0.47 | 0.52 | 0.20 | 0.02 | Bal. | 0.34 | 3113 |
| | A2 | 17.08 | 12.02 | 3.06 | 0.41 | 0.50 | 0.20 | 0.02 | Bal. | 0.42 | 3100 |
| | A3 | 17.88 | 18.33 | 1.90 | 0.56 | 0.81 | 0.04 | 0.11 | Bal. | 0.42 | 3015 |
| Comparative Example | B1 | 18.06 | 1.26 | - | 0.43 | - | 0.07 | 8.44 | Bal. | 0.42 | 2676 |
| | B2 | | | | | | | | | 0.34 | 2873 |

Wire types A1 and A2, which are examples shown in Table 1, are types of wire made of stainless steel, conforming to ASTM F2581. Wire type A3 that is an example is the type of wire made of stainless steel with a large amount of nitrogen and a small amount of carbon compared to those conforming to ASTM F2581.

Wire types B1 and B2 that are comparative examples are types of wire made of SUS304 stainless steel.

The tensile strength of each of wire types, A1-A3, B1, and B2 is the value shown in Table 1. The tensile test is, for example, JIS-Z2241 "Metallic Material-Tensile Testing-Method of test at room temperature". For the wire types, A1, A2, and A3, a plurality of wire materials of each type were subjected to the tensile test, and Table 1 shows the obtained average values of tensile strength.

The wire materials of the wire types in Table 1 were subjected to resilience evaluation, bending evaluation, and rotation performance evaluation.

### <Resilience evaluation>

Wire materials of wire types A1 to A3 that were examples and wire materials of wire types B1 and B2 that were comparative examples were each bent with a jig 2 shown in FIG. 2 and then a residual angle of each wire material was measured.

FIG. 2 is a schematic diagram of the jig 2 to be used for resilience evaluation.

The jig 2 is made of stainless steel, for example, and includes a main body part 3 and a push-in part 4. The main body part 3 has a rectangular parallelepiped shape, and is formed with a push-in groove 3a that opens at two sides. The push-in groove 3a has a substantially rectangular parallelepiped shape, and has, at an end, a push-in recess 3c configured to have a width in the horizontal direction narrower than that of the other part. The push-in part 4 has a rectangular parallelepiped shape and is configured in such a manner that the push-in part 4 can be inserted into the push-in groove 3a. For example, in FIG. 2, the width in the horizontal direction of the push-in groove 3a is 50 mm, the depth of the push-in groove 3a is 1.0 mm, and the depth (width in the vertical direction in FIG. 2) of the push-in recess 3c is 1.5 mm. The width in the horizontal direction of the push-in part 4 is configured to be slightly smaller than the width in the horizontal direction of the push-in hole 3a, and the thickness of the push-in part 4 is 1.0 mm.

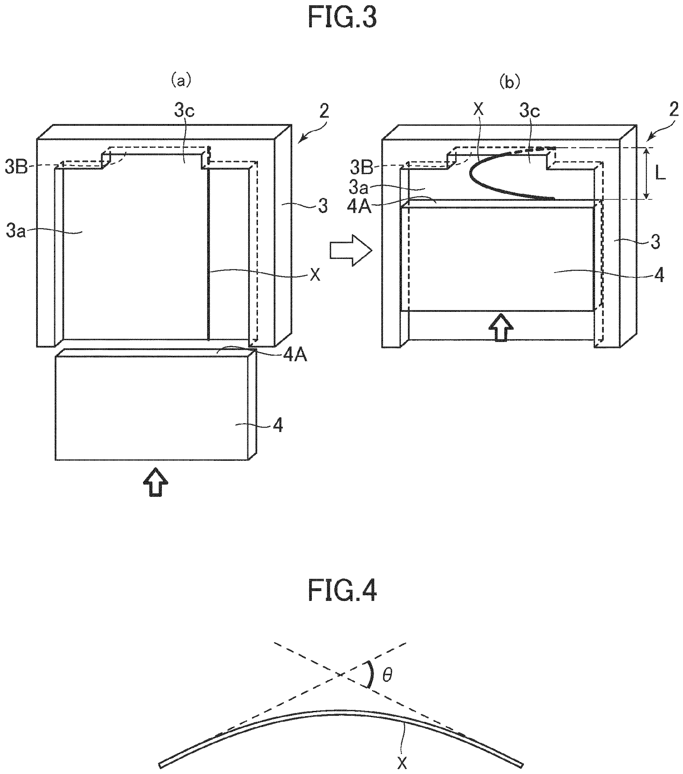

FIG. 3 is an explanatory diagram of resilience evaluation.

As shown in FIG. 3(a), the push-in part 4 is removed from the push-in groove 3a of the main body part 3, and a wire material X cut to have a predetermined length (e.g., 50 mm) is inserted into the push-in groove 3a. At this time, an end of the wire material X are inserted into the push-in recess 3c so as to be brought into contact with an end face 3B forming the push-in recess 3c. As shown in FIG. 3(b), the push-in part 4 is inserted into the push-in groove 3a, and then continuously pushed in, so that the wire material X has a predetermined length (the push-in amount L (mm, the distance between the end face 3B and an end face 4A of the push-in part 4)). Therefore, the wire material X is bent so that its ends approach each other. After keeping the push-in part 4 in the pushed state for about 10 seconds, the push-in part 4 is pulled out from the push-in groove 3a to remove the wire material X. As a result, strain is applied to the entire wire material X. Strain was applied to the wire material X with the push-in amount L (mm) set to 30, 25, 20, 17.5, 15, 12.5, 10, and 7.5. Note that the push-in amount L corresponds to a distance between portions on opposite sides to the sides facing each other at each end when the wire material X is bent so that the ends approach each other.

As shown in FIG. 4, tangent lines are drawn from both ends of the wire material X, an angle at which the two tangent lines intersect (residual angle θ) is measured, and then the resilience rate is calculated. The resilience rate is calculated by (1-θ/180) × 100. Table 2 shows the results of measuring and calculating residual angles and resilience rates.

**[Table 2]**

| | Wire type | | Push-in amount (mm) | | | | | |
|---|---|---|---|---|---|---|---|---|
| | | | 30 | 25 | 20 | 17.5 | 15 | 12.5 |
| Example | A1 | Residual angle (°) | 0 | 0 | 4 | 7 | 13 | 26 |
| | | Resilience rate (%) | 100.0 | 100.0 | 97.7 | 96.3 | 92.7 | 85.6 |
| | A2 | Residual angle (°) | 0 | 4 | 6 | 13 | 25 | 33 |
| | | Resilience rate (%) | 100.0 | 97.9 | 96.5 | 92.8 | 86.3 | 81.8 |
| | A3 | Residual angle (°) | 0 | 4 | 8 | 15 | 21 | 34 |
| | | Resilience rate (%) | 100.0 | 98.0 | 95.8 | 91.9 | 88.1 | 81.0 |
| Comparative Example | B1 | Residual angle (°) | 8 | 12 | 20 | 30 | 41 | 60 |
| | | Resilience rate (%) | 95.7 | 93.5 | 89.1 | 83.5 | 77.3 | 66.7 |
| | B2 | Residual angle (°) | 3 | 4 | 7 | 13 | 20 | 38 |
| | | Resilience rate (%) | 98.3 | 97.6 | 96.0 | 93.1 | 89.1 | 78.9 |

Table 3 shows a strain amount with respect to the push-in amount for each wire diameter. The strain amount (%) is calculated by (R/(L-R)) × 100. Here, R is the wire diameter of each wire material, and L is the push-in amount L (FIG. 3(b)).

**[Table 3]**

| | Push-in amount (mm) | Wire diameter (mm) | |
|---|---|---|---|
| | | 0.42 | 0.34 |
| | | Wire type A2, A3, B1 | Wire type A1, B2 |
| Strain amount (%) | 30 | 1.4 | 1.1 |
| | 25 | 1.7 | 1.4 |
| | 20 | 2.1 | 1.7 |
| | 17.5 | 2.4 | 2.0 |
| | 15 | 2.8 | 2.3 |
| | 12.5 | 3.3 | 2.7 |
| | 10 | 4.2 | 3.4 |
| | 7.5 | 5.6 | 4.6 |

Table 4 lists the resilience rate with respect to the strain amount of each wire type based on Tables 2 and 3. Descriptions of strain amounts of 1.1 or less are omitted.

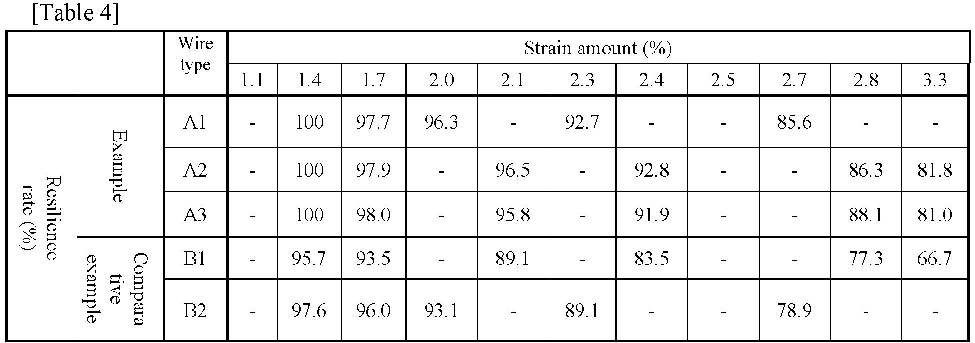

In Table 4, "-" indicates that the measurement was not possible or not performed.

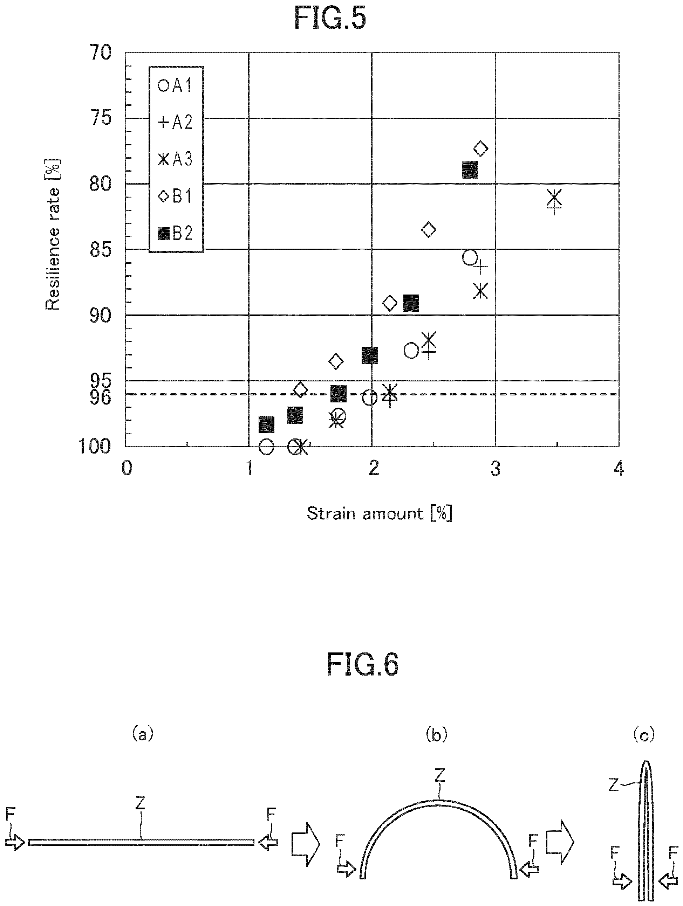

FIG. 5 is a graph that shows changes in the resilience rate with respect to the strain amount of each wire type based on Table 4.

As shown in FIG. 5, the resilience rate of each of the wire types A1 to A3 is 96% or more when the strain amount ranges from 0% to 2%. That is, since the residual angle is smaller than about 7° when the strain amount of 0% to 2% is applied to the wire material, the resilience is good and a decrease in rotational operability can be suppressed in a body lumen.

### <Bending evaluation>

Each wire material of wire types A1 to A3 that were examples and wire types B1 and B2 that were comparative examples was bent about 180° to evaluate the presence or absence of the occurrence of breakage.

FIG. 6 is an explanatory diagram of a bending test.

As shown in FIG. 6(a), force F is applied to both ends of a wire material Z cut to have a predetermined length (for example, 100 mm) to bend the wire material Z as shown in FIG. 6(b), and then as shown in 6(c), the wire material Z is bent about 180° and kept for several seconds (for example, 10 seconds). Then, the presence or absence of the occurrence of breakage at the bent portion of the wire material Z is evaluated.

Table 5 shows the results of evaluating the presence or absence of the occurrence of breakage for each wire type.

**[Table 5]**

| | Wire type | Breakage |
|---|---|---|
| Example | A1 | O |
| | A2 | O |
| | A3 | x |
| Comparative example | B1 | O |
| | B2 | O |

| | | |
|---|---|---|
| Breakage: O No breakage, x With breakage | | |

As shown in Table 5, breakage occurred in wire type A3 that was an example, but breakage did not occur in wire types A1 and A2 that were examples. Therefore, when it is assumed that a wire material to be used as a core shaft of a guide wire is bent with a relatively small radius of curvature, the use of wire types A1 and A2 is more preferable than the use of wire type A3. If a wire material for medical use to be used as the core shaft of a guide wire is expected to be bent with a relatively large radius of curvature, or if a wire material for medical use to be used for purposes other than a guide wire is expected to be bent with a relatively large radius of curvature, any wire material of the examples may be used.

### <Rotational operability evaluation>

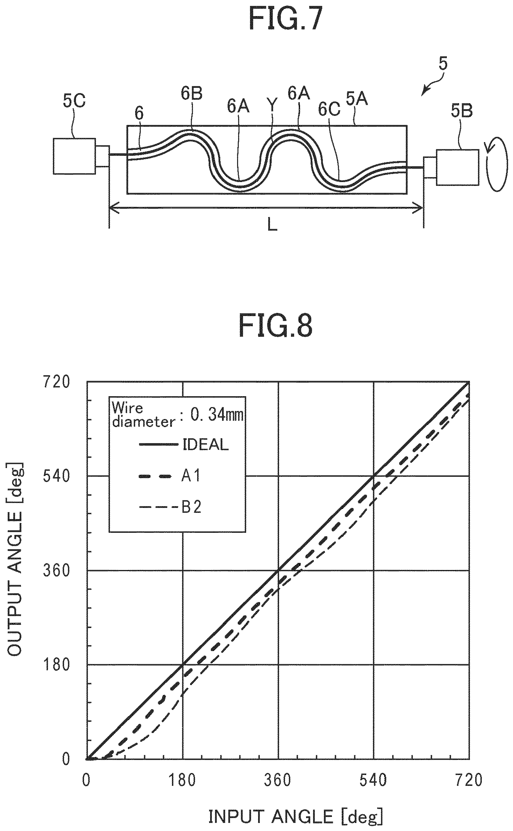

Each wire material of wire types A1 and A2 that were examples and wire types B1 and B2 that were comparative examples was evaluated for rotation performance using the second jig 5 shown in FIG. 7.

FIG. 7 is a schematic diagram of the second jig 5 to be used for evaluating rotation performance.

The second jig 5 includes a wire material insertion part 5A, an input part 5B, and an output part 5C. The wire material insertion part 5A is made of a resin (for example, a transparent acrylic plate) and has a groove 6 formed therein. The groove 6 opens at both ends of the wire material insertion part 5A having substantially rectangular shape. The width of the groove 6 is 1.0 mm. The groove 6 has a plurality of circular arc parts 6A, 6A, 6B, and 6C. Each circular arc part 6A has the same radius of curvature. Circular arc parts 6B and 6C have a radius of curvature larger than the radius of curvature of each circular arc part 6A. In this embodiment, the radius of curvature of the circular arc part 6A is 20 mm. The input part 5B has a substantially columnar shape and is rotatably provided, to which a proximal end of the wire material Y is connected. The output part 5C has a substantially columnar shape and is rotatably provided, to which a distal end of the wire material Y is connected. When the radius of curvature of the circular arc part 6A is 20 mm, the length L between the input part 5B and the output part 5C is configured to be 390 mm. When the radius of curvature of the circular arc part 6A is 20 mm, the length L between the input part 5B and the output part 5C is configured to be 420 mm.

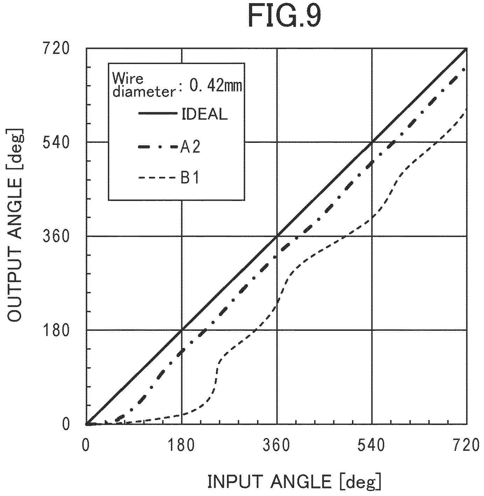

Using the second jig 5, the followability (following angle) of the output part 5C was measured when the input part 5B was rotated clockwise. The results are shown in FIGS. 8 and 9.

FIG. 8 is a graph that shows a relationship between a hand angle and a distal end angle in rotation performance evaluation for wire types A1 and B2 having a wire diameter of 0.34 mm.

FIG. 9 is a graph that shows a relationship between a hand angle and a distal end angle in rotation performance evaluation for wire types A2 and B1 having a wire diameter of 0.42 mm.

As shown in FIG. 8, the wire type A1 that was the example had almost ideal (IDEAL) rotational followability, and the wire type B2 that was the comparative example had initial rotational followability worse than that of the wire type A1 that was the example. As shown in FIG. 9, the wire type A2 that was the example had almost ideal (IDEAL) rotational followability, and the wire type B1 that was the comparative example had overall poor rotational followability. As described above, the wire materials made of stainless steel of wire types A1 and A2 have excellent rotational followability. Since the guide wire 1 of FIG. 1 includes the core shaft 10 made of the stainless steel, it is possible to provide the guide wire 1 having excellent rotational followability.

Note that the disclosure is not limited to the configuration of the above embodiments, but is defined by the terms of the claims and are intended to include any modifications within the scope and meaning equivalent to the terms of the claims.

For example, in the above embodiments, the wire material for medical use is the core shaft 10 of the guide wire 1, but the wire material for medical use may be a wire constituting the coil body 20, or a wire material to be used in other medical instruments, such as a wire that constitutes a reinforcing body (for example, a tubular mesh body, a coil body) to be used for reinforcing a catheter, for example. The coil body 20 or the reinforcing body of a catheter is configured using the wire material for medical use of the above embodiments, so that it is possible to improve the resilience of the coil body 20 or the reinforcing body after bending. In addition, medical members configured of coils and hollow bodies produced using the wire material become possible to exhibit excellent rotational followability in the same manner as described above when rotational operation is performed in intricate blood vessels.

### [Reference Signs List]

1 Guide wire
10 Core shaft

## Claims

1. A wire material for medical use made of stainless steel, having a resilience rate of 96% or more after a strain amount of 2% is applied by bending the wire material so that each end approaches each other, wherein the strain amount (%) = (R/(L-R)) × 100 (R is a wire diameter of the wire material, L is a distance between both ends when the wire material is bent so that each end approaches each other), and the resilience rate (%) = (1-θ/180) × 100 (θ is an angle at which two tangent lines intersect when the tangent lines are drawn from both ends of the wire material after bending).

2. The wire material for medical use for medical use according to claim 1, wherein the stainless steel has tensile strength of 2800 N/mm² or more and less than 3400 N/mm².

3. The wire material for medical use according to claim 1, wherein the stainless steel is austenitic stainless steel conforming to ASTM F2581.

4. The wire material for medical use according to any one of claim 1 to claim 3, wherein no breakage occurs at a bent portion when the wire material is bent 180°.

5. A guide wire, comprising the wire material for medical use according to any one of claims 1 to 4 and a coil body provided around the wire material for medical use.
